# EUROPEAN PATENT SPECIFICATION

(11) **EP 1 432 681 B1**
(45) Date of publication and mention of the grant of the patent: **08.08.2007**
(21) Application number: 02777139.3
(22) Date of filing: 18.09.2002
(51) Int. Cl.: C07D 209/88, A61P 9/00

(54) **PSEUDOPOLYMORPHIC FORMS OF CARVEDILOL**
PSEUDOPOLYMORPHE FORMEN VON CARVEDILOL
FORMES PSEUDOPOLYMORPHES DE CARVEDILOL

(30) Priority: 28.09.2001 EP 01123422
(43) Date of publication of application: 30.06.2004
(73) Proprietor: F. HOFFMANN-LA ROCHE AG, 4070 Basel (CH)
(72) Inventor: BUBENDORF, Andre Gerard, F-68510 Uffheim (FR); GABEL, Rolf-Dieter, D-68723 Schwetzingen (DE); HENNING, Michael, D-79576 Weil am Rhein (DE); KRIMMER, Siegfried, D-79585 Steinen (DE); NEUGEBAUER, Guenter, 68309 Mannheim (DE); PREIS, Walter, D-67433 Neustadt (DE); WIRL, Alexander, D-67259 Heuchelheim (DE)
(74) Representative: Heiroth, Ulrike Hildegard
(86) International application number: PCT/EP2002/010451
(87) International publication number: WO 2003/029214

(56) References cited:
- EP-A- 0 893 440
- WO-A-02/00216

## Description

The present invention relates to pseudopolymorphic forms of (±)1-(4-carbazolyloxy)-3-[2-(2-methoxyphenoxy)ethylamino]-2-propanole (carvedilol) as well as of optically active forms or pharmaceutically acceptable salts thereof. The present invention also relates to processes for the preparation of such pseudopolymorphic forms of carvedilol and to pharmaceutical compositions containing them.

Carvedilol is a non-selective β-blocker with a vasodilating component, which is brought about by antagonism to the α₁-adrenoreceptors. Moreover, carvedilol also has antioxidative properties. Carvedilol is the object of European Patent No. 0 004 920 and can be manufactured according to the processes described therein.

Carvedilol has a chiral center and, as such, can exist either as individual stereoisomers or in racemic form. Both the racemate and stereoisomers may be obtained according to procedures well known in the art (EP-B-0127099).

WO 99/05105 discloses a thermodynamically stable modification of carvedilol with a melting point of 123-126°C (hereinafter referred to as carvedilol form I), compared to the carvedilol described in EP 0 004920 having a melting point of 114-115°C (hereinafter referred to as carvedilol form II).

At pH values in the pharmaceutically relevant range of 1 to 8 the solubility of carvedilol in aqueous media lies between about 1 mg and 100 mg per 100 ml (depending on the pH value). This has been found to be problematical especially in the formulation of highly concentrated parenteral formulations, such as e.g. injection solutions or other formulations for the production of small volume administration forms for ocular or oral administration.

In the case of the peroral administration of rapid release carvedilol formulations, e.g. the commercial formulation, resorption quotas of up to 80% are achieved, with a considerable part of the resorbed carvedilol being very rapidly metabolized.

In connection with investigations into the gastrointestinal resorption of carvedilol it has been established that the resorption of carvedilol becomes poorer during the course of passage through the gastrointestinal tract and e.g. in the ileum and colon makes up only a fraction of the resorption in the stomach. This has been found to be very troublesome especially in the development of retard forms in which a release should take place over several hours. The poorer resorption is presumably due entirely or at least in part to the decreasing solubility of carvedilol with increasing pH values. A very low solubility can also be established in the strongly acidic region (about pH 1-2).

In order to improve the resorption quota, especially in the lower regions of the intestine, investigations have been carried out for adjuvants and, respectively, formulations which are suitable for increasing the solubility and/or speed of dissolution of carvedilol.

Accordingly, one underlying purpose of the invention lay in improving the resorption of carvedilol, especially in the case of peroral administration and here especially in the lower regions of the intestine, using agents available in pharmaceutical technology.

It now has surprisingly been found that the pseudopolymorphic forms of (±)1-(4-carbazolyloxy)-3-[2-(2-methoxyphenoxy)ethylamino]-2-propanole (carvedilol) according to the present invention, especially the hydrates of carvedilol, particularly carvedilol hemihydrate (hereinafter referred to as form IV), can be formulated at high concentrations in a composition further comprising certain selected adjuvants. Such compositions containing carvedilol form IV have a better active substance resorption and thus an improved bioavailability compared with formulations which contain carvedilol forms I or II.

Carvedilol can thus be isolated in different modifications depending upon the method of preparation. The three polymorphic forms are monotropic and distinguishable by their infrared and X-ray powder diffraction spectra and their melting point.

In one embodiment, the present invention provides a new crystalline modification (form IV) of carvedilol substantially free of other physical forms, having a melting point of approximately T Onset 94-96°C measured by Differential Scanning Calorimetry. The IR spectrum of form IV shows great differences in the stretching vibration range (3526, 3492 and 3400 cm⁻¹) compared to the spectra of forms I and II. The X-ray powder diffraction pattern of carvedilol form IV has characteristic peaks occurring at 2θ = 7.0, 8.3, 11.5, 15.7, and 17.2.

As used herein, the term "pseudopolymorphic forms" relates to hydrates and solvates, preferably to hydrates. Pseudopolymorphic forms of carvedilol, such as hydrates and solvates, contain different amounts of water or solvents in the crystal lattice.

The term "hydrates" encompasses compounds with different amounts of water present in the crystal lattice, such as hemi hydrates, monohydrates, dihydrates, with hemihydrates being especially preferred.

"Pharmaceutically acceptable salts" of carvedilol embrace alkali metal salts, such as Na or K salts, alkaline earth metal salts, such as Ca and Mg salts, as well as salts with organic or inorganic acids, such as, for example, hydrochloric acid, hydrobromic acid, nitric acid, sulphuric acid, phosphoric acid, citric acid, formic acid, maleic acid, acetic acid, succinic acid, tartaric acid, methanesulphonic acid or toluenesulphonic acid, which are non-toxic for living organisms.

For the resolution of the racemates, there can be used for example tartaric acid, malic acid, camphoric acid or camphorsulphonic acid.

Where reference is made in this application to carvedilol form I, II and IV substantially free of other physical forms, it means that at least 75% by weight, preferably 90% by weight, more preferable 95% by weight of carvedilol form I, II or IV, respectively, is present in the preparation.

Pseudopolymorphic forms of carvedilol, i.e. hydrates and solvates, can generally be prepared by crystallisation out of solvents in which carvedilol is soluble, for example alcohols, such as methanol, ethanol and isopropanol, acetone, acetonitrile, chloroform, dimethylformamide, dimethylsulfoxide, methylenechloride or mixtures thereof or with water.

Furthermore, crystalline form IV of carvedilol can be prepared by isolation of form IV from spray congealed material of carvedilol, the preparation of which is described below, followed by re-crystallisation in methanol/water. Thus, carvedilol form IV was first isolated from spray congealed material prepared according to Example 4 of WO 01/74357. Furthermore, using carvedilol form II as starting material, seeding with carvedilol form IV ensures the crystallisation of form IV.

In a further aspect, the present invention provides pharmaceutical compositions comprising a pseudopolymorphic form of carvedilol form IV substantially free of other physical forms of carvedilol, a pharmaceutically acceptable carrier and/or adjuvant and, if desired, other active ingredients. Such compositions may be used for the treatment or prophylaxis of illnesses.

The compounds of the present invention may be administered by any suitable route, preferably in the form of a pharmaceutical composition adapted to such a route and in dose effective for the treatment intended. The compounds and compositions may, for example, be administered orally, intravascularly, intraperitoneally, subcutaneously, intramuscularly or topically. Preferred mode of administration is oral administration. The pharmaceutical composition may be in the form of, for example, a tablet, capsule, creme, ointment, gel, lotion, suspension or liquid. The pharmaceutical composition is preferably made in the form of a dosage unit containing a particular amount of the active ingredient. Examples of such dosage units are tablets or capsules.

Therapeutically effective doses of the compounds of the present invention required to prevent or arrest the progress of the medical condition are readily ascertained by one of ordinary skill in the art. The dose regimen for treating a disease condition with the compounds and/or compositions of this invention is selected in accordance with a variety of factors, including the type, age, weight, sex and medical conditions of the patient and in accordance to the severity of the disease and thus may vary widely. A suitable daily dose for a mammal may vary widely depending on the condition of the patient and other factors. However, a dose from about 0.01 to 1.00 mg/kg body weight, particularly from about 0.05 to 3 mg/kg body weight, respectively 0.01 to 10 mg/cm² skin, may be appropriate. The active ingredient may also be administered by injection.

For therapeutic purposes, the compounds of the invention are ordinarily combined with one or more adjuvants appropriate to the indicated route of administration. If per os, the compound may be mixed with lactose, sucrose, starch powder, cellulose esters of alkanoic acids, cellulose alkyl ester, talc, stearic acid, magnesium stearate, magnesium oxide, sodium and calcium salts of phosphoric and sulphuric acids, gelatine, acacia, sodium alginate, polyvinyl-pyrrolidone and/or polyvinyl alcohol, and thus tabletted or encapsulated for convenient administration. Alternatively, the compound may be dissolved in water, polyethylene glycol, propylene glycol, ethanol, corn oil, cotton seed oil, peanut oil, sesame oil, benzyl alcohol, sodium chloride and/or various buffers. Appropriate additives for the use as ointments, cremes or gels are for example paraffin, vaseline, natural waxes, starch, cellulose, or polyethylenglycole (PEG). Other adjuvants and modes of administration are well and widely known in the pharmaceutical art.

Preferable pharmaceutical compositions containing a pseudopolymorphic form of carvedilol form IV, can be prepared with selected adjuvants which are not surface-active, such as polyethylene glycols (PEG) or sugar substitutes as well as non-ionic tensides, such as polyoxyethylene stearates, e.g. Myrj^{®} 52, or polyoxyethylene-polyoxypropylene copolymers, e.g. Pluronic^{®} F 68.

The content of hydrophilic polyoxyethylene groups in the aforementioned polyoxyethylene-polyoxypropylene copolymers preferably lies at 70% to 90%. In an especially preferred embodiment the ratio of hydrophilic polyoxyethylene groups to hydrophobic polyoxypropylene groups lies at about 80:20 and the average molecular weight preferably lies at about 8,750.

The aforementioned polyoxyethylene stearates preferably have a hydrophilic-lipophilic balance (HLB) value of 10 to 20, preferably of 14 to 20, especially of 16 to 18.

From the series of sugar substitutes especially isomalt (hydrogenated isomaltulose), e.g. Palatinit^{®}, has been found to be particularly suitable. Palatinit^{®} is a hydrogenated isomaltulose, which consists of about equal parts of 1-*O*-α-D-glucopyranosyl-D-sorbitol and 1-*O*-α-D-glucopyranosyl-D-mannitol dihydrate.

Further, in connection with the present invention polyethylene glycols with a molecular weight of 200 to 20,000, preferably 1,000 to 20,000, more preferably 4,000 to 10,000, particularly 6,000 to 8,000, have been found to be especially suitable.

In a preferred embodiment of the present invention the carvedilol form I, II or IV is dissolved in a non-ionic tenside, preferably Pluronic^{®} F 68, Or in an adjuvant which is not surface-active, preferably polyethylene glycol 6,000.

Thus, carvedilol form I, II or IV can be dissolved in polyethylene glycol 6,000 which is melted at about 70°C. In this manner there are obtained highly concentrated compositions of carvedilol (up to 500 mg/ml). Moreover, further additives, for example cellulose derivatives such as hydroxypropylmethylcelluloses or hydroxypropylcelluloses, can be admixed in order to control the release rate of the active substance. Further, the compositions in accordance with the invention can contain highly dispersed silicon dioxide as an anti-caking agent.

In a preferred embodiment, the carvedilol form IV content in the compositions in accordance with the invention lies at 5% (wt./wt.) to 60% (wt./wt.), preferably at 5% (wt./wt.) to 50% (wt./wt.), especially at 10% (wt./wt.) to 40% (wt./wt.), with the weight % details relating to the total weight of the composition (active substance and adjuvant).

In a preferred embodiment the adjuvants in accordance with the invention have a melting point below 120 °C, especially a melting point of 30 °C to 80 °C.

The aforementioned adjuvants can be used individually or in a combination of two or more adjuvants with one another. The combination of an adjuvant which is not surface-active, preferably polyethylene glycol, with a non-ionic tenside, preferably a polyoxyethylene-polyoxypropylene copolymer, e.g. Pluronic^{®} F 68, is especially preferred, since the addition of surface-active substances can accelerate the active substance release from the composition.

Compositions of carvedilol form IV which contain as adjuvants polyethylene glycol, preferably polyethylene glycol 6,000, as well as 0.1% to 50%, preferably 0.1% to 10%, of polyoxyethylene-polyoxypropylene copolymers, e.g. Pluronic^{®} F 68, have been found to be especially suitable.

In a particular embodiment of the present invention the ratio of the aforementioned adjuvant which is not surface-active, for example polyethylene 6,000, to the surface-active adjuvant, for example Pluronic^{®} F 68, lies between 1000:1 and 1:1, preferably between 100:1 and 10:1.

The compositions of carvedilol form IV in accordance with the invention and medicaments produced therefrom can contain further additives such as, for example, binders, plasticizers, diluents, carrier substances, glidants, antistatics, antioxidants, adsorption agents, separation agents, dispersants, dragéeing laquer, de-foamers, film formers, emulsifiers, extenders and fillers.

The aforementioned additives can be organic or inorganic substances, e.g. water, sugar, salts, acids, bases, alcohols, organic polymeric compounds and the like. Preferred additives are lactose, saccharose, tablettose, sodium carboxymethylstarch, magnesium stearate, various celluloses and substituted celluloses such as, for example, methylhydroxy-propylcellulose, polymeric cellulose compounds, highly dispersed silicon dioxide, maize starch, talcum, various polymeric polyvinylpyrrolidone compounds as well as polyvinyl alcohols and their derivatives. It is a prerequisite that all additives used in the production are non-toxic and advantageously do not change the bioavailability of the active substance

In a preferred embodiment the compositions in accordance with the invention contain carvedilol form IV in a substantially pure form, polyethylene glycol, polyoxyethylene-polyoxypropylene copolymer as well as highly dispersed silicon dioxide. In an especially preferred embodiment the compositions in accordance with the invention contain 10-20% (wt./wt.) carvedilol form IV, 65-85% (wt./wt.) polyethylene glycol, 1-10% (wt./wt.) polyoxyethylene-polyoxypropylene copolymer and 0.1-10% (wt./wt.) highly dispersed silicon dioxide, with the percentages relating to the total weight of the four named substances irrespective of whether additional adjuvants are present in the composition.

The compositions of carvedilol form IV in adjuvants can be prepared by dissolving carvedilol form I, II or IV in the molten adjuvants, followed by rapid solidification of the melt of the adjuvants with the dissolved active substance, e.g. by spray congealing. Alternatively, the compositions of carvedilol form IV in adjuvants can be prepared by dissolving the polymer carrier (PEG) in an appropriate organic solvent or solvent mixture (e.g. ethanol, methanol, isopropanol, acetonitrile, aceton or mixtures thereof and/or with water), followed by the addition of carvedilol form I, II or IV. Thereafter, the solvent is removed by spray drying. Storage at room temperature for about 1 to 2 months results in a composition containing carvedilol in substantially pure form IV as an active ingredient. Depending on the conditions used in the spray solidification step, formation of form IV can be achieved within one week to several months.

The present invention is therefore also concerned with a process for the production of compositions of carvedilol form IV, which comprises the admixture of carvedilol with molten hydrophilic adjuvants, such as, for example, polyethylene glycol, and/or surface-active substances, such as, for example, Pluronic^{®}F 68. Alternatively, the active compound and adjuvants may be mixed with subsequent melting. In a preferred embodiment the thus-obtained formulation is subsequently spray congealed.

In the case of spray drying, the material to be dried is sprayed as a solution or suspension at the upper end of a wide, cylindrical container through an atomizer arrangement to give a droplet mist. The resulting droplet mist is mixed with hot air (preferably > 100°C) or an inert gas which is conducted into the dryer around the atomization zone. The resulting solvent vapour is taken up by the drying air and transported away, and the separated powder is removed from the container via a separator.

In the case of spray congealing, the material to be solidified is sprayed as a melt at the upper end of a wide, cylindrical container through a heatable atomizer arrangement to give a droplet mist. The resulting droplet mist is mixed with cooled air (preferably < 25°C), which is conducted into the dryer around the atomization zone. The heat of congealing which is liberated is taken up by the air and transported away, and the separated solidified powder is removed from the container via a separator. As atomizer arrangements there come into consideration (heatable) pressure nozzles (e.g. pressure nozzle with swirl bodies), pneumatic nozzles (binary/ternary nozzles) or centrifugal atomizers.

The resulting compositions of carvedilol form IV can be advantageously used pharmaceutically in various ways. Thus, for example, such compositions can be processed further to rapid release administration forms, such as, for example, tablets, film tablets, capsules, granulates, pellets, etc. with an improved resorption quotient. This permits under certain circumstances a dosage reduction in comparison with conventional rapid release peroral medicaments which have been produced using crystalline carvedilol form II.

The resulting compositions of carvedilol form IV can also be used especially advantageously for the production of medicaments with a modified release characteristic. Under a modified release characteristic there is to be understood, for example, a 95% release after more than two hours, preferably after 2 to 24 hours, or a pH-dependent release in which the beginning of the release is delayed in time. For this purpose, the carvedilol compositions can be processed to or with all conventional pharmaceutical oral medicaments with modified release.

Examples of medicaments with a modified release characteristic are film tablets which are resistant to gastric juice or retard forms, such as e.g. hydrocolloid matrices or similar medicaments from which the active substance is released via an erosion or diffusion process. The formulations in accordance with the invention can be processed to formulations with modified active substance release by the addition of further adjuvants or film coatings or by incorporation in conventional pharmaceutical release systems. Thus, the formulations in accordance with the invention can be incorporated, for example, in hydrocolloid matrix systems, especially in those which are based on cellulose derivatives such as hydroxypropylcellulose, hydroxypropylmethylcellulose, methylcellulose or polyacrylate derivatives such as, for example, Eudragit RL. The aforementioned matrices can contain, additionally or alternatively, a hydrocolloid matrix former which swells depending on pH, such as, for example, sodium alginate or sodium carboxymethylcellulose. By the addition of such an adjuvant a targeted release which is individually determined can be achieved. Thereby, the use of the compositions in accordance with the invention leads to an appreciable improvement in the resorption in comparison to the crystalline carvedilol form IV as active substance.

Thus, the spray congealed compositions of carvedilol in accordance with the invention, preferably those comprising Pluronic^{®} F 68, polyethylene glycol 6000, highly dispersed silicon dioxide and carvedilol (preferably in accordance with Example 4), can be pressed to tablets, for example, by direct compression, granulation and compacting together with hydrophilic matrix formers which control the release, such as e.g. hydroxypropylmethylcelluloses 2208 with an average viscosity of about 100 mPa • s (Methocel^{®} K100 LV-Premium) and hydroxypropylmethylcelluloses 2208 with an average viscosity of about 4000 mPa • s (Methocel^{®} K4M-Premium), and with glidants or anti-caking agents, such as e.g. magnesium stearate and microcrystalline celluloses (Avicel^{®} PH102). Moreover, the tablets can be coated with a conventional lacquer, such as e.g. Opadry^{®} II White Y-30-18037 and Opadry^{®} Clear YS-1-7006.

The pharmaceutical compositions in accordance with the invention are suitable for the production of conventional pharmaceutical administration forms, preferably oral administration forms, for the treatment and/or prophylaxis of cardiac and circulatory disorders, such as e.g. hypertension, cardiac insufficiency and angina pectoris.

The dosage in which the pharmaceutical compositions in accordance with the invention are administered depends on the age and the requirements of the patients and the route of administration. In general, for oral administration, single dosages of about 0.1 mg to 50 mg of carvedilol per day come into consideration. For this, formulations with a carvedilol active substance content of about 1 mg to 50 mg are used.

The present invention is therefore also concerned with a method for the treatment of illnesses, such as hypertension, cardiac insufficiency or angina pectoris, which comprises the administration of medicaments which contain the pharmaceutical formulations described above.

### Characterization of Form IV of Carvedilol

### Differential Scanning Calorimetry (DSC)

DSC (Differential Scanning Calorimetry) was carried out on a Mettler TA 8000 system with a DSC 821e, a sample robot and intracooler equipment. Dry nitrogen was used as purge gas (flow 150 ml/min) and dry gas (flow 150 ml/ min). The scan rates were 5°C/min and 1°C/min (heating and cooling cycles) and the sample weigh ranging from 1 to 12 mg. Sealable 40µl aluminum pans hermetically closed with a perforation lid were used. Prior to measurement the lid was automatically pierced resulting in approx. 1.5 mm pin holes. All measurements were performed with pierced lids. Calibration of temperature and heat of fusion was performed with 99.999% indium (Mettler-Toledo (Schweiz) AG; CH-Greifensee). Melting point 156.6 °C; Heat of fusion 28.45 J/g.

The measured melting point (T Onset) of carvedilol form IV was about 94-96°C. Heat of fusion of carvedilol form IV was ΔHf 144-154J/g corresponding to 60-64 kJ/mol for the hemihydrate (molecular weight: 406.5 + 9).

TGA (Thermal Gravimetric Analysis) was carried out on a Mettler TA 8000 system with a TGA 851e and a sample robot and air cooling. Dry nitrogen was used as purge gas (flow 50 ml/min) and dry gas (flow 20 ml/ min). The scan rates were 5°C/min and 1°C min (heating and cooling cycles), the sample weigh ranging from 10 to 50 mg. Sealable 100 µl aluminum pans hermetically closed with a perforation lid were used. Prior to measurement the lid was automatically pierced resulting in approx. 1.5 mm pin holes. Sealed pans prevent any exchange of solvents and humidity with the atmosphere during the waiting position in the sample robot.

The determined weight loss (weight step) between 50°C and 140°C was approximately 2.2 % (weight percent), corresponding to ½ mole of water for the molecular weight of the hemihydrate.

### FT-IR and X-ray diffractometry

The IR-spectrum of the sample is recorded as film of a Nujol suspension consisting of approx. 15 mg of sample and approx. 15 mg of Nujol between two sodium chloride plates, with an FT-IR spectrometer in transmittance. The Spectrometer is a Nicolet 20SXB or equivalent (resolution 2 cm-1, 32 or 64 coadded scans, MCT detector).

X-ray powder diffraction was carried out with a Stoe X-ray diffractometer STADIP in transmission, Cu_{KαI}-radiation, Ge-monochromator, rotation of sample during measurement, position sensitive detector (PSD), angular range 2° to 32° (2θ), steps of 0.5° (2θ), measuring time 40 seconds per step.

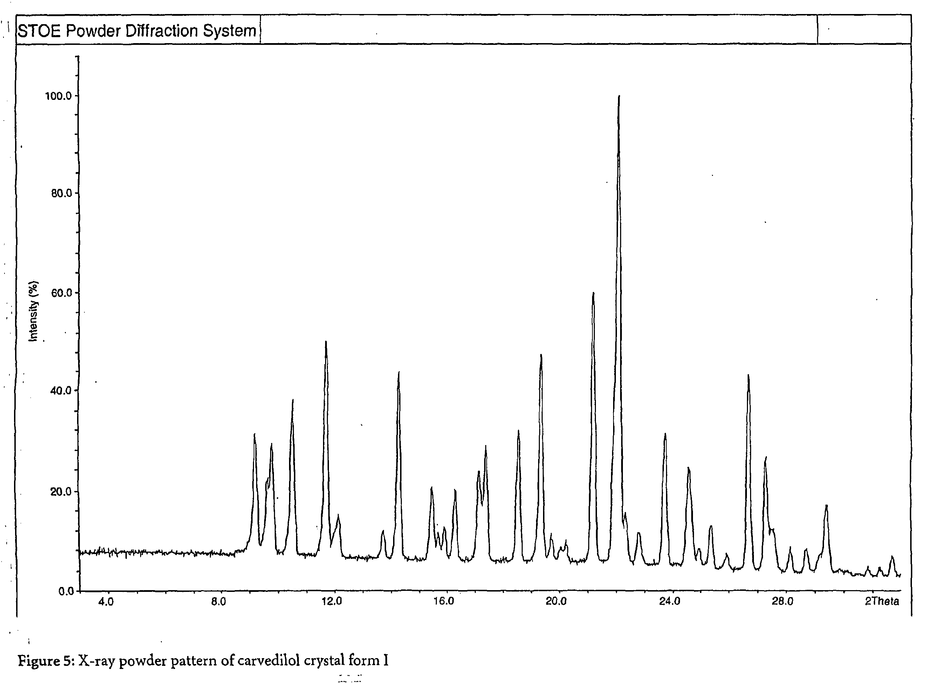

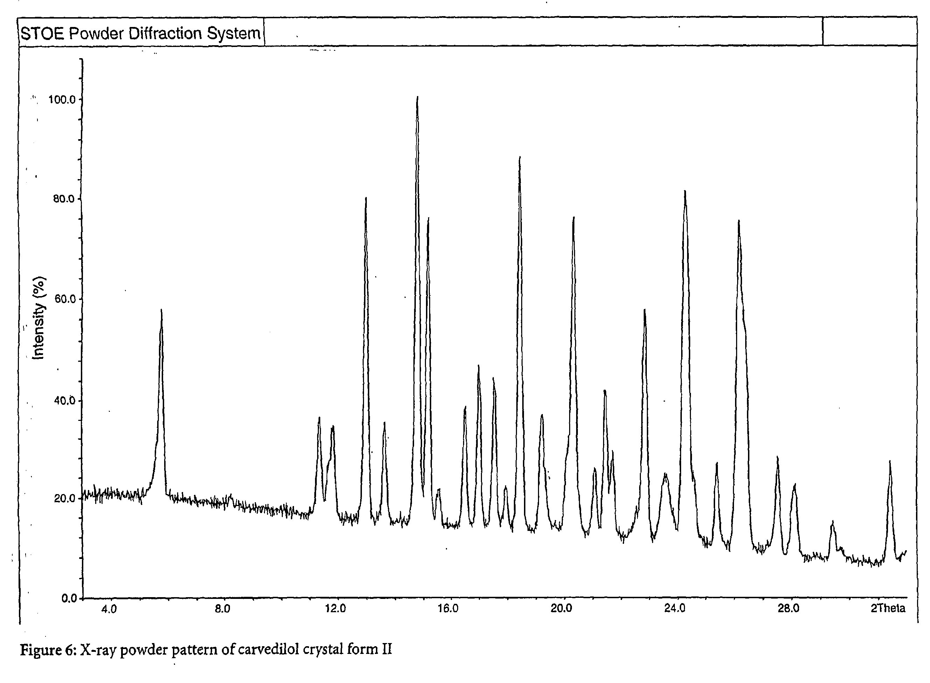

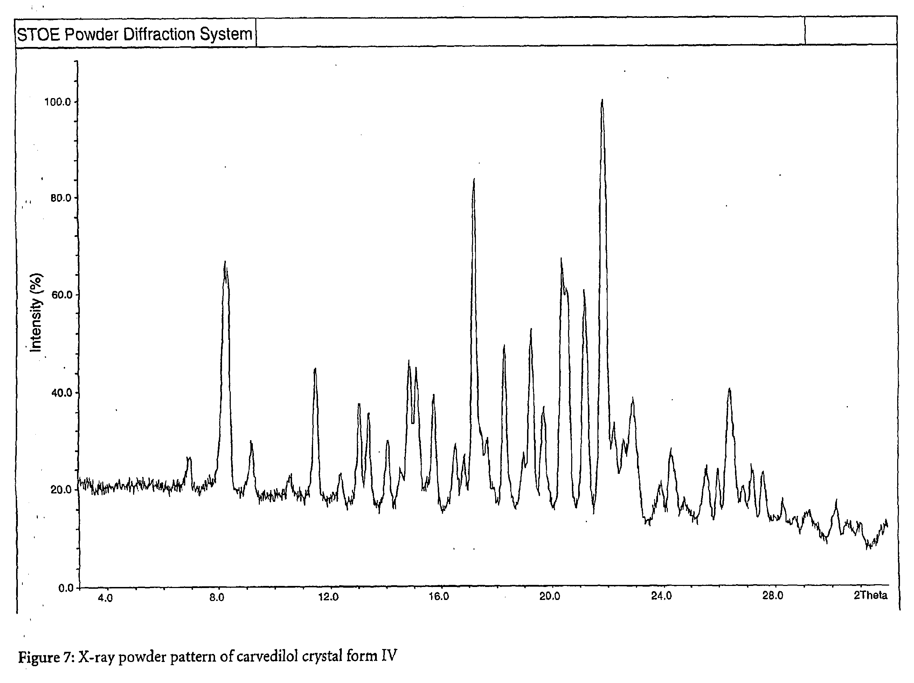

The X-ray powder diffraction pattern of form IV has characteristic peaks at 2θ = 7.0°, 8.3° (is subdivided in two peaks at 8.235° + 8.383°), 11.5°, 15.7°, and 17.2° (Figure 5). Characteristic peaks of the form II occur at 2θ = 5.9°, 14.9°, 17.6°, 18.5°, and 24.4° (Figure 6) and of the form I at 10.5°, 11.7°, 14.3°, 18.5°, 19.3°, 21.2°, 22.1° (Figure 7).

Crystal data for C₂₄H₂₆N₂O₄* C₂₄H₂₆N₂O₄*H₂O (two molecules carvedilol and one water molecule), monoclinic space group P2₁/n, a=13.517(3)Å, b=16.539(3)Å, c=19.184(4)Å, β=94.27(3)°, V=4276.9(15) Å³, Z=8, Data were recorded on a STOE image plate detector using Mok_{α} (graphite monochromator) radiation, a colourless crystal of dimensions 0.3x0.3x0.05mm was used and a total of 5298 unique measurements collected. The structure was solved using direct methods and refined to a R_{factor} of 0.0764. There are two molecules of carvedilol and one water molecule in the asymmetric unit of the crystal. The theoretical X-ray powder diffraction pattern calculated from this structure coincides well with the experimentally derived X-ray powder diffraction pattern of samples of crystal form IV.

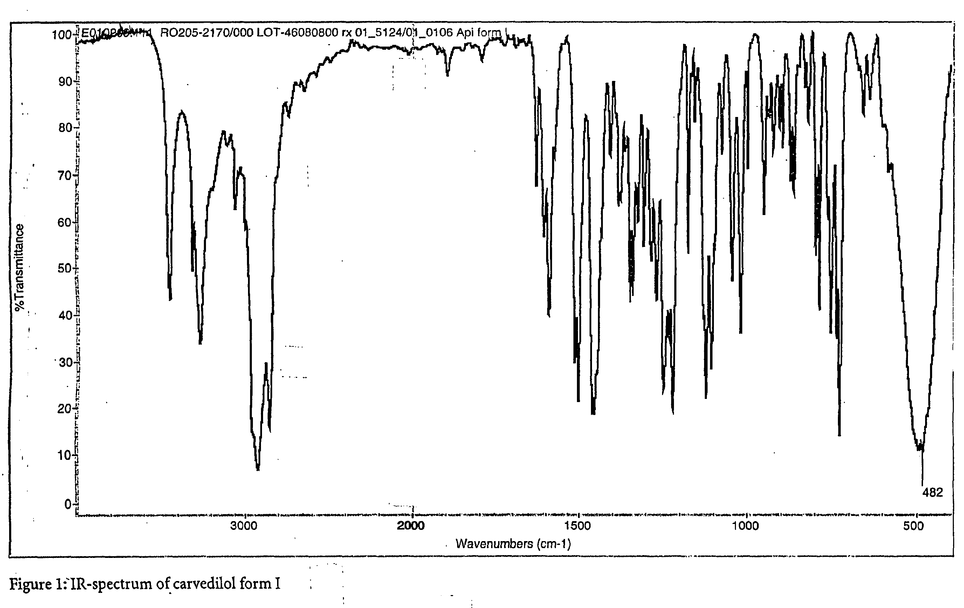

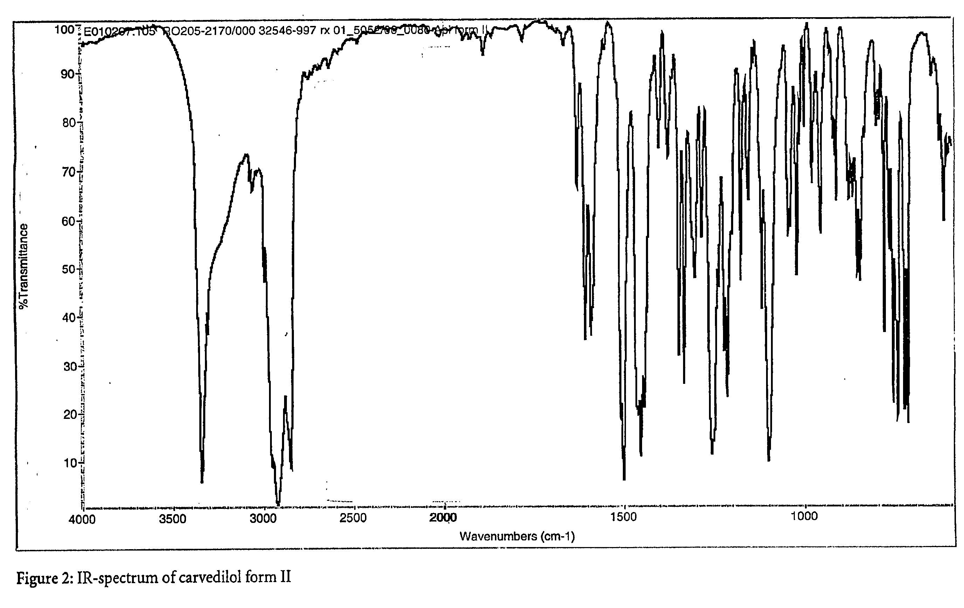

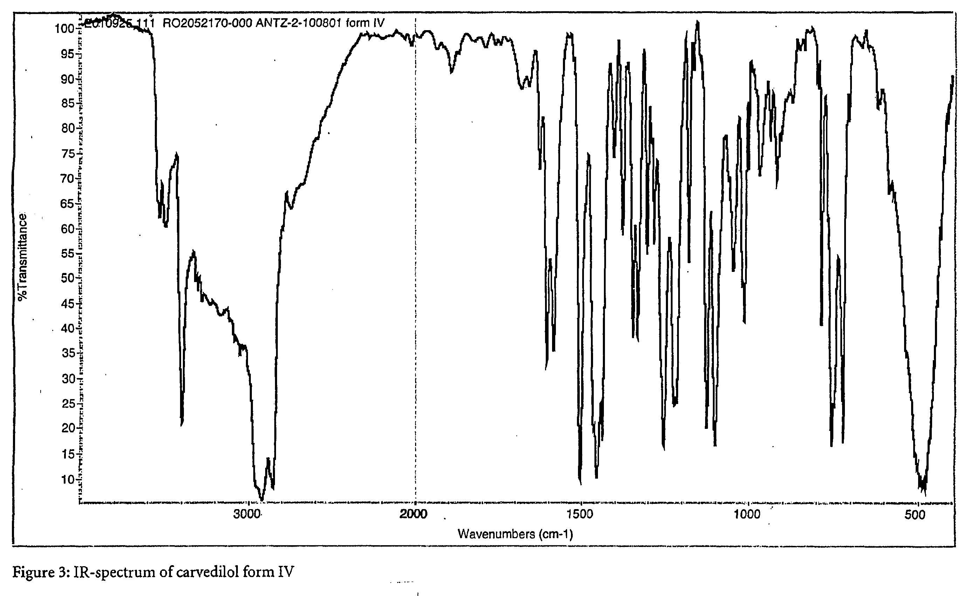

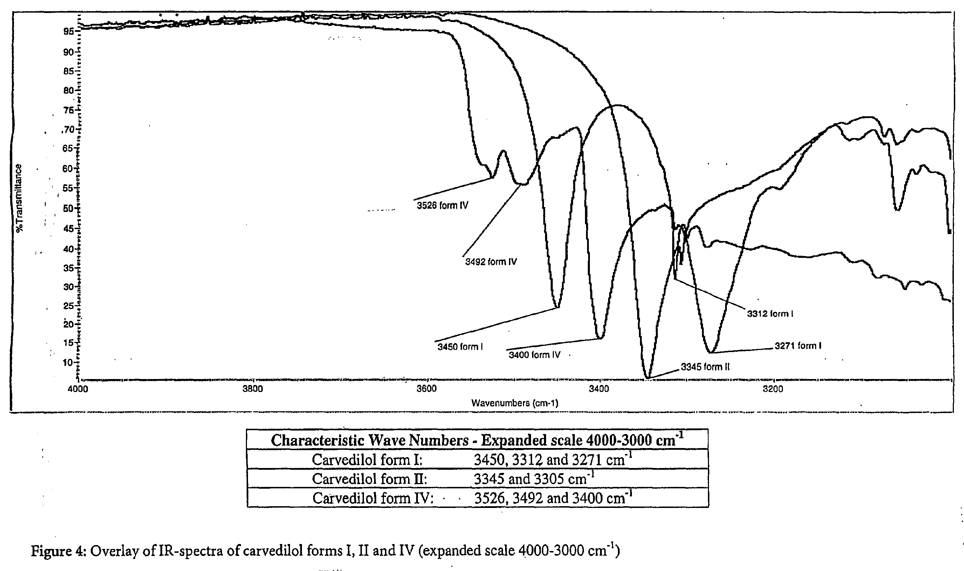

The IR spectrum of carvedilol form IV shows the biggest differences compared to the spectra of carvedilol forms I and II in the stretching vibration range form IV 3400 cm⁻¹; form I 3450 cm⁻¹; form II 3345 cm⁻¹ (see Figures 1 to 4), which are caused by different hydrogen bridges.

The following Examples are intended to describe the preferred embodiments of the present invention, without thereupon limiting this.

### Example 1

### Preparation of spray congealed carvedilol

The spray congealed carvedilol used to isolate form IV was prepared according the following procedure: Macrogol 6000 (polyethylene glycol) is first molten at 70 to 85°C. Subsequent dissolution of Pluronic F 68 (polypropylene glycol) and carvedilol form II at 70 to 85°C yields a melt with the following composition (batch size: approximately 10 kg): 16.84% carvedilol; 5.05% Pluronic F68 and 78.11% Macrogol 6000.

This melt is spray congealed using cold nitrogen (0 to 5°C) via a heated two-fluid nozzle. The spray congealed material is collected using a cyclone separator. Prior to further use the batch is stored at 4 to 8 °C for 8 month.

### Example 2

### Process for preparing carvedilol form IV

9g of spray congealed carvedilol and 100 ml of distilled water are stirred over night at RT with a magnetic stirrer. The obtained suspension is filtered through a 0.45 µm filter and washed two times with 20 ml of distilled water. The filter cake is re-suspended in 100 ml of distilled water and stirred again over night. The so obtained suspension is again filtered through a 0.45 µm filter, washed two times with 20 ml of distilled water and dried in vacuum (10-15 mbar) at RT for at least 12 hours to yield approximately 1.6 g of form IV. The obtained form IV is characterised as described before.

To obtain pure form IV, 130 mg of the above isolated material is suspended in 3.25 ml methanol/water (90:10 v/v) and heated up to 50-60°C until all material is dissolved. The solution is cooled down to RT during one hour and stored overnight at RT. The so obtained crystalline material is isolated and dried in a dry nitrogen stream to yield 70-100 mg of pure crystalline form IV. The obtained form IV is characterised as described before.

To obtain bigger crystals of form IV for X-Ray single crystal measurements, 100 mg of the above isolated material is suspended in 4 ml methanol/water (90:10 v/v) and heated up to 50°C-60°C until all material is dissolved. The solution is cooled down very slow from 55°C to minus 10°C during 50 hours. The so obtained crystalline material is isolated and dried in dry nitrogen stream to yield 50-80 mg of pure crystalline form IV. These obtained crystals were usable to perform X-Ray single crystal measurements.

### Example 3

### Process for preparing carvedilol form IV

118 mg of carvedilol form II is suspended in 3 ml methanol/water (90:10 v/v) and heated up to 50-60°C until a clear solution is obtained. The solution is cooled down to 40-50°C and seeded with a small amount of crystallised form IV (obtained as described in Example 2). The seeded solution is cooled down to RT and stored over night at 5-8°C. The so obtained crystalline material is isolated and dried in dry nitrogen stream to yield 50-80 mg of pure crystalline form IV. The obtained form IV is characterised as described before.

### Example 4

Composition containing carvedilol form IV

| | |
|---|---|
| Carvedilol | 50.0 g |
| Polyethylene glycol 6,000 | 250.0 g |
| Total weight: | 300.0 g |

The polyethylene glycol 6,000 is melted at 70 °C. The carvedilol form II is stirred into the resulting melt and homogeneously dissolved. Then, the melt is spray congealed. Alternatively, the melt can be solidified by means of other methods, provided that the solidification takes place rapidly. Storage at room temperature for about 2 months results in a composition containing carvedilol in substantially pure form IV as an active ingredient.

### Example 5

Composition containing carvedilol form IV

| | |
|---|---|
| Carvedilol | 50.0 g |
| Polyethylene glycol 6,000 | 250.0 g |
| Total weight: | 300.0 g |

The polyethylene glycol 6,000 is melted at 70 °C. The carvedilol form I is stirred into the resulting melt and homogeneously dissolved. Then, the melt is spray congealed. Alternatively, the melt can be solidified by means of other methods, provided that the solidification takes place rapidly. Storage at room temperature for about 2 months results in a composition containing carvedilol in substantially pure form IV as an active ingredient.

### Example 6

Composition containing carvedilol form IV

| | |
|---|---|
| Carvedilol | 50.0 g |
| Polyoxyethylene-polyoxypropylene copolymer | 250.0 g |
| Total weight: | 300.0 g |

The polyoxyethylene-polyoxypropylene copolymer is melted at 70 °C. The carvedilol form II is stirred into the resulting melt and homogeneously dissolved. Then, the melt is spray congealed. Alternatively, the melt can be solidified by means of other methods, provided that the solidification takes place rapidly. Storage at room temperature for about 2 months results in a composition containing carvedilol in substantially pure form IV as an active ingredient.

### Example 7

Composition containing carvedilol form IV

| | |
|---|---|
| Carvedilol | 50.0 g |
| Polyoxyethylene-polyoxypropylene copolymer | 15.0 g |
| Polyethylene glycol 6,000 | 235.0 g |
| Total weight: | 300.0 g |

The polyethylene glycol 6,000 is melted at 70 °C. Subsequently, the polyoxyethlene-polyoxypropylene copolymer is stirred into the above melt, likewise melted and the melt is homogenized. The carvedilol form II is stirred into the resulting melt and homogeneously dissolved. Then, the melt is spray congealed. Alternatively, the melt can be solidified by means of other methods, provided that the solidification takes place rapidly. Storage at room temperature for about 2 months results in a composition containing carvedilol in substantially pure form IV as an active ingredient.

If desired, the technical processing properties such as, for example, the flowability of the solutions can be improved by the addition of further adjuvants, see Example 9.

### Example 8

Composition containing carvedilol form IV

| | |
|---|---|
| Carvedilol | 50.0 g |
| Polyoxyethylene-polyoxypropylene copolymer | 15.0 g |
| Polyethylene glycol 6,000 | 235.0 g |
| Total weight: | 300.0 g |

The polyethylene glycol 6,000 is melted at 70 °C. Subsequently, the polyoxyethlene-polyoxypropylene copolymer is stirred into the above melt, likewise melted and the melt is homogenized. The carvedilol form I is stirred into the resulting melt and homogeneously dissolved. Then, the melt is spray congealed. Alternatively, the melt can be solidified by means of other methods, provided that the solidification takes place rapidly. Storage at room temperature for about 2 months results in a composition containing carvedilol in substantially pure form IV as an active ingredient.

If desired, the technical processing properties such as, for example, the flowability of the solutions can be improved by the addition of further adjuvants, see Example 9.

### Example 9

Composition containing carvedilol form IV

| | |
|---|---|
| Carvedilol | 50.0 g |
| Polyoxyethylene-polyoxypropylene copolymer | 15.0 g |
| Polyethylene glycol 6,000 | 232.0 g |
| Silicon dioxide, highly dispersed | 3.0 g |
| Total weight: | 300.0 g |

The polyethylene glycol 6,000 is melted at 70 °C. Subsequently, the polyoxyethlene-polyoxypropylene copolymer is stirred into the above melt, likewise melted and the melt is homogenized. The carvedilol form II is stirred into the resulting melt and homogeneously dissolved. Then, the melt is spray congealed. Alternatively, the melt can be solidified by means of other methods, provided that the solidification takes place rapidly. The carvedilol composition is then treated with highly dispersed silicon dioxide and mixed homogeneously. Storage at room temperature for about 2 months results in a composition containing carvedilol in substantially pure form IV as an active ingredient.

### Example 10

Composition containing carvedilol form IV

| | |
|---|---|
| Carvedilol | 50.0 g |
| Polyoxyethylene-polyoxypropylene copolymer | 125.0 g |
| Polyethylene glycol 6,000 | 125.0 g |
| Total weight: | 300.0 g |

The polyethylene glycol 6,000 is melted at 70 °C. Subsequently, the polyoxyethlene-polyoxypropylene copolymer is stirred into the above melt, likewise melted and the melt is homogenized. The carvedilol form II is stirred into the resulting melt and homogeneously dissolved. Then, the melt is spray congealed. Alternatively, the melt can be solidified by means of other methods, provided that the solidification takes place rapidly. Storage at room temperature for about 2 months results in a composition containing carvedilol in substantially pure form IV as an active ingredient.

### Example 11

Composition containing carvedilol form IV

| | |
|---|---|
| Carvedilol | 50.0 g |
| Isomalt | 450.0 g |
| Total weight: | 500.0 g |

The isomalt is melted at above its melting point. Subsequently, the carvedilol form II is stirred into the resulting melt and homogeneously dissolved. Then, the melt is spray congealed. Alternatively, the melt can be solidified by means of other methods, provided that the solidification takes place rapidly. Storage at room temperature for about 2 months results in a composition containing carvedilol in substantially pure form IV as an active ingredient.

### Example 12

Composition containing carvedilol form IV - Rapid release tablets:

| | |
|---|---|
| Carvedilol | 50.0 g |
| Polyoxyethylene-polyoxypropylene copolymer | 15.0 g |
| Polyethylene glycol 6,000 | 232.0 g |
| Silicon dioxide, highly dispersed | 3.0 g |
| Tablettose | 146.0 g |
| Sodium carboxymethylstarch | 15.0 g |
| Silicon dioxide, highly dispersed | 4.0 g |
| Magnesium stearate | 10.0 g |
| Total weight: | 475.0 g |

The polyethylene glycol 6,000 is melted at 70 °C. Subsequently, the polyoxyethlene-polyoxypropylene copolymer is stirred into the above melt, likewise melted and the melt is homogenized. The carvedilol form II is stirred into the resulting melt and homogeneously dissolved. Then, the melt is spray congealed. Alternatively, the melt can be solidified by means of other methods, provided that the solidification takes place rapidly. The mixture is subsequently treated with highly dispersed silicon dioxide and mixed homogeneously. The mixture obtained is treated with tablettose and mixed. The outer phase (lubricant, flow agent, separating agent and extender) consisting of sodium carboxymethylstarch, highly dispersed silicon dioxide and magnesium stearate is added to the above mixture and mixed homogeneously. The resulting mixture is then pressed to pharmaceutical forms or filled into capsules in the usual manner taking into consideration the desired active substance content. Storage at room temperature for about 2 months results in a composition containing carvedilol in substantially pure form IV as an active ingredient.

### Example 13

Composition containing carvedilol form IV - Retard tablets:

| | |
|---|---|
| Carvedilol | 50.0 g |
| Polyoxyethylene-polyoxypropylene copolymer | 15.0 g |
| Polyethylene glycol 6,000 | 232.0 g |
| Silicon dioxide, highly dispersed | 3.0 g |
| Tablettose | 146.0 g |
| Hydroxypropylmethylcellulose 2208 | 240.0 g |
| Silicon dioxide, highly dispersed | 4.0 g |
| Magnesium stearate | 10.0 g |
| Total weight: | 700.0 g |

The polyethylene glycol 6,000 is melted at 70 °C. Subsequently, the polyoxyethlene-polyoxypropylene copolymer is stirred into the above melt, likewise melted and the melt is homogenized. The carvedilol form II is stirred into the resulting melt and homogeneously dissolved. Then, the melt is spray congealed. Alternatively, the melt can be solidified by means of other methods, provided that the solidification takes place rapidly. The mixture is subsequently treated with highly dispersed silicon dioxide and mixed homogeneously. The mixture obtained is treated with tablettose and mixed. The outer phase (lubricant, flow agent, separating agent and extender), consisting of hydroxypropylmethylcellulose 2208, highly dispersed silicon dioxide and magnesium stearate is added to the above mixture and mixed homogeneously. The resulting mixture is then pressed to pharmaceutical forms or filled into capsules in the usual manner taking into consideration the desired active substance content. Storage at room temperature for about 2 months results in a composition containing carvedilol in substantially pure form IV as an active ingredient.

### Example 14

Composition containing carvedilol form IV - Retard tablets:

| | |
|---|---|
| Carvedilol | 50.0 g |
| Polyoxyethylene-polyoxypropylene copolymer | 15.0 g |
| Polyethylene glycol 6,000 | 232.0 g |
| Silicon dioxide, highly dispersed | 3.0 g |
| Tablettose | 96.0 g |
| Hydroxypropylmethylcellulose 2208 | 240.0 g |
| Sodium alginate | 50.0 g |
| Silicon dioxide, highly dispersed | 4.0 g |
| Magnesium stearate | 10.0 g |
| Total weight: | 700.0 g |

The polyethylene glycol 6,000 is melted at 70 °C. Subsequently, the polyoxyethlene-polyoxypropylene copolymer is stirred into the above melt, likewise melted and the melt is homogenized. The carvedilol form II is stirred into the resulting melt and homogeneously dissolved. Then, the melt is spray congealed. Alternatively, the melt can be solidified by means of other methods, provided that the solidification takes place rapidly. The carvedilol composition is subsequently treated with highly dispersed silicon dioxide and mixed homogeneously. The mixture obtained is treated with tablettose and mixed. The outer phase (lubricant, flow agent, separating agent and extender), consisting of sodium alginate, highly dispersed silicon dioxide and magnesium stearate is added to the above mixture and mixed homogeneously. The resulting mixture is then pressed to pharmaceutical forms or filled into capsules in the usual manner taking into consideration the desired active substance content.

## Claims

1. Pseudopolymorphic form of (±)1-(4-carbazolyloxy)-3-[2-(2-methoxyphenoxy)ethylamino]-2-propanole hemihydrate having the following X-ray diffraction pattern obtained with a Cu_{KαI}-radiation at 2θ = 7.0, 8.3,11.5,15.7, and 17.2, an infrared spectrum having sharp peaks at 3526 cm⁻¹, 3492 cm⁻¹ and 3400 cm⁻¹, and a melting point of approximately T Onset = 94-96°C.

2. The use of a compound of claim 1 for the manufacture of a medicament for the treatment or prophylaxis of cardiac diseases.

3. A pharmaceutically acceptable composition comprising a compound of claim 1 and a pharmaceutically acceptable carrier and/or adjuvant

4. The composition according to claim 3, wherein one or more adjuvants in the composition are not surface-active.

5. The composition according to claim 4, wherein polyethylene glycol is present as the adjuvant which is not surface-active.

6. The composition according to claim 5, wherein the polyethylene glycol has a molecular weight of 200 to 20,000, preferably 4,000 to 10,000.

7. The composition according to any one of claims 3 to 6, wherein a sugar substitute is present as the adjuvant which is not surface-active.

8. The composition according to claim 7, wherein isomalt is present as the sugar substitute.

9. The composition according to any one of claims 3 to 8, wherein one or more non-ionic tensides are present.

10. The composition according to claim 9, wherein a polyoxyethylene-polyoxypropylene copolymer is present as the non-ionic tenside.

11. The composition according to any of claims 9 to 10, wherein a polyoxyethylene stearate is present as the non-ionic tenside.

12. The composition according to any of claims 9 to 11, wherein the ratio of adjuvants which are not surface active to non-ionic tensides lies between 1000:1 and 1:1, preferably between 100:1 and 10:1.

13. The composition according to any of claims 3 to 12, wherein the compound of any of claims 1 to 4 is present in a concentration between 5% (wt./wt.) and 60% (wt./wt.).

14. The composition according to claim 13, wherein the compound of claim 1 is present in a concentration between 10% (wt./wt.) and 40% (wt./wt.).

15. The composition according to any of claims 3 to 14, wherein highly dispersed silicon dioxide is present.

16. A composition according to any of claims 3 to 15, which contains 10-20% (wt./wt.) of a compound of claim 1,65-85% (wt./wt.) polyethylene glycol, 1-10% (wt./wt.) polyoxyethylene-polyoxypropylene copolymer and 0.1-10% (wt./wt.) highly dispersed silicon dioxide.

17. A pharmaceutically acceptable administration form comprising a composition according to any of claims 3 to 16.

18. A pharmaceutically acceptable administration form according to claim 17, which has a modified active substance release, with 95% of the active substance being released in 2 to 24 hours.

19. A pharmaceutically acceptable administration form according to any of claims 17 to 18, which is a solid administration form.

20. A pharmaceutically acceptable administration form according to any of claims 17 to 19, which is an oral administration form.

21. A process for the production of a composition according to any one of claims 3 to 16, which process comprises melting a compound of claim 1 in an adjuvant which is not surface-active and/or a non-ionic tenside.

22. The process according to claim 21, wherein the resulting melt is solidified by spray congealing.

23. The use of a composition according to any of claims 3 to 16 for the production of a medicament for the treatment or prophylaxis of illnesses such as hypertension, cardiac insufficiency or angina pectoris.

## Patentansprüche

1. Pseudopolymorphe Form von (±)1-(4-Carbazolyloxy)-3-[2-(2-methoxyphenoxy)ethylamino]-2-propanol-Hemihydrat mit folgendem Röntgenbeugungsdiagramm, erhalten mit Cu_{KαI}-Strahlung bei 2θ = 7,0, 8,3, 11,5, 15,7 und 17,2, einem Infrarotspektrum mit scharfen Banden bei 3526 cm⁻¹, 3492 cm⁻¹ und 3400 cm⁻¹, und einem Schmelzpunkt von ungefähr T Onset = 94-96°C.

2. Verwendung einer Verbindung von Anspruch 1 für die Herstellung eines Medikaments zur Behandlung oder Prophylaxe von Herzkrankheiten.

3. Pharmazeutisch verträgliche Zusammensetzung, umfassend eine Verbindung von Anspruch 1 und einen pharmazeutisch verträglichen Träger und/oder Hilfsstoff.

4. Zusammensetzung gemäß Anspruch 3, wobei ein oder mehrere Hilfsstoffe in der Zusammensetzung nicht oberflächenaktiv sind.

5. Zusammensetzung gemäß Anspruch 4, wobei Polyethylenglycol als nicht-oberflächenaktiver Hilfsstoff enthalten ist.

6. Zusammensetzung gemäß Anspruch 5, wobei das Polyethylenglycol ein Molekulargewicht von 200 bis 20.000, vorzugsweise 4.000 bis 10.000 besitzt.

7. Zusammensetzung gemäß einem der Ansprüche 3 bis 6, wobei ein Zuckerersatzstoff als nicht-oberflächenaktiver Hilfsstoff enthalten ist.

8. Zusammensetzung gemäß Anspruch 7, wobei Isomalt als der Zuckerersatzstoff enthalten ist.

9. Zusammensetzung gemäß einem der Ansprüche 3 bis 8, wobei eines oder mehrere nichtionische Tenside enthalten sind.

10. Zusammensetzung gemäß Anspruch 9, wobei ein Polyoxyethylen-Polyoxypropylencopolymer als nicht-ionisches Tensid enthalten ist.

11. Zusammensetzung gemäß einem der Ansprüche 9 bis 10, wobei ein Polyoxyethylenstearat als nicht-ionisches Tensid enthalten ist.

12. Zusammensetzung gemäß einem der Ansprüche 9 bis 11, wobei das Verhältnis von Hilfsstoffen, die nicht oberflächenaktiv sind, zu nicht-ionischen Tensiden zwischen 1.000 : 1 und 1 : 1, vorzugsweise zwischen 100 : 1 und 10 : 1, liegt.

13. Zusammensetzung gemäß einem der Ansprüche 3 bis 12, wobei die Verbindung eines der Ansprüche 1 bis 4 in einer Konzentration zwischen 5% (Gew./Gew.) und 60% (Gew./Gew.) enthalten ist.

14. Zusammensetzung gemäß Anspruch 13, wobei die Verbindung von Anspruch 1 in einer Konzentration zwischen 10% (Gew./Gew.) und 40% (Gew./Gew.) enthalten ist.

15. Zusammensetzung gemäß einem der Ansprüche 3 bis 14, wobei hochdisperses Siliciumdioxid enthalten ist.

16. Zusammensetzung gemäß einem der Ansprüche 3 bis 15, enthaltend 10-20% (Gew./Gew.) einer Verbindung von Anspruch 1, 65-85% (Gew./Gew.) Polyethylenglycol, 1-10% (Gew./Gew.) Polyoxyethylen-Polyoxypropylencopolymer und 0,1 bis 10% (Gew./Gew.) hochdisperses Siliciumdioxid.

17. Pharmazeutisch verträgliche Darreichungsform, umfassend eine Zusammensetzung gemäß einem der Ansprüche 3 bis 16.

18. Pharmazeutisch verträgliche Darreichungsform gemäß Anspruch 17, die eine modifizierte Wirkstofffreisetzung, bei der 95% des Wirkstoffes innerhalb von 2 bis 24 Stunden freigesetzt werden, aufweist.

19. Pharmazeutisch verträgliche Darreichungsform gemäß einem der Ansprüche 17 bis 18, welche eine feste Darreichungsform ist.

20. Pharmazeutisch verträgliche Darreichungsform gemäß einem der Ansprüche 17 bis 19, welche eine orale Darreichungsform ist.

21. Verfahren zur Herstellung einer Zusammensetzung gemäß einem der Ansprüche 3 bis 16, wobei das Verfahren das Schmelzen einer Verbindung aus Anspruch 1 in einem nichtoberflächenaktiven Hilfsstoff und/oder einem nicht-ionischen Tensid umfasst.

22. Verfahren gemäß Anspruch 21, wobei die resultierende Schmelze durch Sprüherstarren verfestigt wird.

23. Verwendung einer Zusammensetzung gemäß einem der Ansprüche 3 bis 16 zur Herstellung eines Medikaments zur Behandlung oder Prophylaxe von Krankheiten wie Bluthochdruck, Herzinsuffizienz oder Angina pectoris.

## Revendications

1. Forme pseudo-polymorphe d'hémihydrate de (±)1-(4-carbazolyloxy)-3-[2-(2-méthoxyphénoxy)éthyl-amino]-2-propanol ayant le diagramme de diffraction des rayons X suivant obtenu avec un rayonnement de Cu_{KαI} à 2θ = 7,0, 8,3, 11, 5, 15,7 et 17,2, un spectre à infrarouge ayant des pics nets à 3526 cm⁻¹, 3492 cm⁻¹ et 3400 cm⁻¹ et un point de fusion correspondant approximativement à T_{début} = 94-96°C.

2. Utilisation d'un composé de la revendication 1, pour la production d'un médicament destiné au traitement ou à la prophylaxie de maladies cardiaques.

3. Composition pharmaceutiquement acceptable comprenant un composé de la revendication 1 et un support et/ou adjuvant pharmaceutiquement acceptable.

4. Composition suivant la revendication 3, dans laquelle un ou plusieurs adjuvants dans la composition sont non tensioactifs.

5. Composition suivant la revendication 4, dans laquelle du polyéthylèneglycol est présent comme adjuvant qui est non tensioactif.

6. Composition suivant la revendication 5, dans laquelle le polyéthylèneglycol a un poids moléculaire de 200 à 20 000, de préférence de 4000 à 10 000.

7. Composition suivant l'une quelconque des revendications 3 à 6, dans laquelle un produit de substitution de sucre est présent comme adjuvant qui est non tensioactif.

8. Composition suivant la revendication 7, dans laquelle de l'isomalt est présent comme un produit de substitution de sucre.

9. Composition suivant l'une quelconque des revendications 3 à 8, dans laquelle un ou plusieurs agents tensioactifs non ioniques sont présents.

10. Composition suivant la revendication 9, dans laquelle un copolymère polyoxyéthylène-polyoxypropylène est présent comme agent tensioactif non ionique.

11. Composition suivant l'une quelconque des revendications 9 et 10, dans laquelle un poly(stéarate d'oxyéthylène) est présent comme agent tensioactif non ionique.

12. Composition suivant l'une quelconque des revendications 9 à 11, dans laquelle le rapport des adjuvants qui sont non tensioactifs aux agents tensioactifs non ioniques est de 1000:1 à 1:1, de préférence de 100:1 à 10:1.

13. Composition suivant l'une quelconque des revendications 3 à 12, dans laquelle le composé de l'une quelconque des revendications 1 à 4 est présent à une concentration de 5 % (en poids/poids) à 60 % (en poids/poids).

14. Composition suivant la revendication 13, dans laquelle le composé de la revendication 1 est présent à une concentration de 10 % (en poids/poids) à 40 % (en poids/poids).

15. Composition suivant l'une quelconque des revendications 3 à 14, dans laquelle du dioxyde de silicium hautement dispersé est présent.

16. Composition suivant l'une quelconque des revendications 3 à 15, qui contient 10 à 20 % (en poids/poids) d'un composé de la revendication 1, 65 à 85 % (en poids/poids) de polyéthylèneglycol, 1 à 10 % (en poids/poids) d'un copolymère polyoxyéthylène-polyoxypropylène et 0,1 à 10 % (en poids/poids) de dioxyde de silicium hautement dispersé.

17. Forme d'administration pharmaceutiquement acceptable comprenant une composition suivant l'une quelconque des revendications 3 à 16.

18. Forme d'administration pharmaceutiquement acceptable suivant la revendication 17, qui présente une libération de substance active modifiée, une proportion de 95 % de la substance active étant libérée en 2 à 24 heures.

19. Forme d'administration pharmaceutiquement acceptable suivant l'une quelconque des revendications 17 et 18, qui est une forme d'administration solide.

20. Forme d'administration pharmaceutiquement acceptable suivant l'une quelconque des revendications 17 à 19, qui est une forme d'administration orale.

21. Procédé pour la production d'une composition suivant l'une quelconque des revendications 3 à 16, procédé qui comprend la fusion d'un composé de la revendication 1 dans un adjuvant qui n'est pas tensioactif et/ou dans un agent tensioactif non ionique.

22. Procédé suivant la revendication 21, dans lequel la masse fondue résultante est solidifiée par figeage par atomisation.

23. Utilisation d'une composition suivant l'une quelconque des revendications 3 à 16 pour la production d'un médicament destiné au traitement ou à la prophylaxie de maladies telles que l'hypertension, l'insuffisance cardiaque ou l'angine de poitrine.
